# EUROPEAN PATENT SPECIFICATION

(11) **EP 1 750 575 B1**
(45) Date of publication and mention of the grant of the patent: **05.06.2013**
(21) Application number: 05741888.1
(22) Date of filing: 10.05.2005
(51) Int. Cl.: A61B 5/00, A61B 8/08

(54) **APPARATUS FOR EXAMINING A BODY OF LIVING TISSUES**
GERÄT ZUR UNTERSUCHUNG EINES KÖRPERS MIT LEBENDEM GEWEBE
DISPOSITIF SERVANT A EXAMINER UNE MASSE DE TISSUS VIVANTS

(30) Priority: 10.05.2004 GB 0410320; 08.02.2005 GB 0502524
(43) Date of publication of application: 14.02.2007
(73) Proprietor: Highland Innovation Centre Limited, Inverness, IV3 8NE (GB)
(72) Inventor: WATMOUGH, David, John, Inverness IV3 8NE (GB)
(74) Representative: Macdonald, Kate
(86) International application number: PCT/GB2005/001777
(87) International publication number: WO 2005/107577

(56) References cited:
- WO-A-03/077744
- WO-A2-2004/080291
- US-A1- 2003 004 499
- US-A1- 2003 032 950
- US-B1- 6 264 610
- US-B1- 6 290 699

## Description

The present invention relates to an apparatus for examining a body of living tissues, in particular the female breast or male testicular tissues.

The principal health concern of women is breast cancer. Why? Because it is the commonest cause of death in women under 50 years of age. In the UK there are 41,000 new cases per year and the trend is upwards. There are 13,000 deaths per year from breast cancer. The 5 year survival is 73%. This means 1 in 4 women die within 5 years of diagnosis. The appropriate management of patients with early disease reduces mortality. Presently 15% of women present with advanced disease. Earlier detection should also reduce the number of mastectomies in favour of breast conserving surgery. What can be done to ensure that women with the disease present at an early stage for diagnosis and treatment?

Similar problems concern testicular cancer in men.

The present applicant has developed an easy to use, light-based product, the breast checker, which when employed together with breast self examination is designed to indicate the need for women to consult a GP for advice and possible referral to a hospital setting. We refer to our co-pending patent applications UK 0105471.7 [published as GB 2375672A] and International Patent Application No. PCT /GB/02/01159 [published as WO 03/077744A].

The applicant's prior breast checker is small, safe, simple to use and is purposely chosen to be non-diagnostic. It involves no ionising radiation, no UV radiation, or breast compression. The applicant has also developed a more sophisticated version of the breast checker for use by GPs which employs an optical/ultrasound Doppler probe. Errors sometimes lead to claims for compensation. The price of such failure can exceed £300,000. The clinical breast checker should minimise false reassurance and thus cut the error rate.

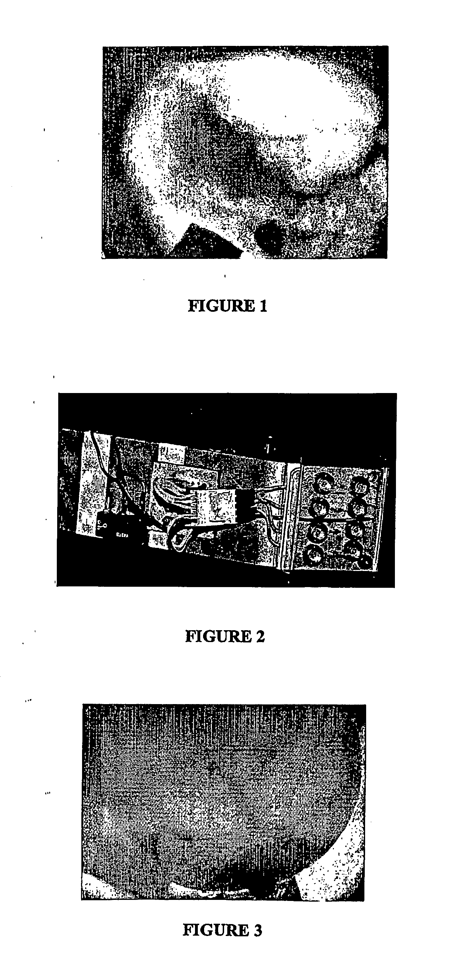

A typical image of an advanced breast cancer is shown by the dark area in the accompanying Figure 1. The fact that angiogenesis is responsible for the excess light absorption is supported by the presence of the connected dilated vessels seen to the right of the nipple [small dark circle].

Figure 1 shows an advanced cancer of the breast (arrowed) obtained by means of light transmitted through the tissues from a light guide held against the inferior breast surface.

This present invention aims to provide improvements to the breast checker instruments described in our earlier co-pending applications. In those applications the light source was envisaged as typically a quartz halogen 75 Watt bulb which requires a current of about 6 amperes at 12 volts and thus necessitates a cooling fan since the device can be switched on for extended periods. This increases the minimum size of the device thus requiring separate probe and control box. For prolonged use in a clinical setting mains power is the only practical option. The direct use of mains voltage [240 Volts] in the probe containing the light source is considered undesirable in an instrument in contact with the human body. The instruments described here are all used in a darkened room so that variations in brightness on the surface of a breast through which light is transmitted may be observed.

Ideally a totally self contained hand held unit powered by means of typically 3 or 4 AA type rechargeable batteries is desirable but difficult to implement in practice. The unit requires to provide light of sufficient intensity to transmit a few percent (of that light incident upon it) through an essentially conical tissue volume and to thus provide as nearly as possible, uniform illumination on the exit side [usually the superior aspect of the breast]. Non uniform illumination prevents detection of brightness variations indicative of the presence of small deep malignant tumours. Without use of a tissue compression system, [which is particularly undesirable for incorporation into a domestic device because of both cost and safety] the breast generally approximates to a conical volume, the apex being centred on the nipple.

The problem therefore arises as to how to achieve such uniform brightness over the breast surface in normal breasts (where there is no neoplastic tumour present).

The applicant initially tried a number of light emitting diodes, [LEDs] first in a 4 x 4 array as shown in figure 2. The preferred LEDs are 22 Lumens per Watt high intensity devices.

Figure 2 shows a first arrangement of 8 LEDs used to demonstrate the possibility of illuminating breast tissues from the inferior aspect with these multiple light sources.

Figure 3 shows an image of a breast obtained using apparatus shown in Figure 2 showing serious backscatter of light below and around the periphery of the breast.

With 8 1 Watt LEDs as shown in Figure 2 there is excessive scatter of lights laterally, medially and below the breast. This will prevent a woman using the breast checker from seeing variations in brightness on the superior aspect of her breast. It is still a problem if she uses a mirror to observe her breasts while applying the breast checker.

The present invention aims to overcome this problem.

US-A-2003/0004499 discloses systems for the photomodulation of living tissue. The systems include LEDs but are not concerned with the examination of living tissues, in particular the female breast.

US 2003/0032950 describes a cooling system for a photo cosmetic device. Radiation is delivered to the tissue via optical systems designed to pattern the radiation and project the radiation to a particular depth. Contact sensors and motion sensors may be used to enhance treatment.

The present invention provides a handheld battery powered apparatus having the features of the appended claims. Accordingly, the present invention provides a handheld battery powered apparatus for examining a body of living tissues, the apparatus comprising:
a light source for illuminating the tissues;
the light source comprising a substantially D shaped array of high intensity light emitting diodes (1-7;1-8;1-5,8,9) overlaid by an optically transparent window (8,9;10) wherein said light emitting diodes (1-7;1-8;1-5,8,9) operate at a wavelength lying between 550 and 700 nm;
a control means for reproducing the intensity of the light from the light source operarable to allow repeatability of the light intensity used in one examination to be reproduced in the next;
characterised in that
the apparatus comprises a pressure or capacitive sensor whereby the light level is held at a low level until the pressure or capacitive sensor touches the skin of the body of tissues whereupon the light level increases to operate at a preset level of light intensity.

Preferably the apparatus comprises means (6) for applying ultrasound to a neoplasm to be examined, means (7) for receiving reflected ultrasound from blood vessels in the tissues, and a loudspeaker or other means for generating an output to be analysed by a user which reproduces blood flow signals derived from the reflected ultrasound signals.

Preferably, the means for applying ultrasound and means for receiving reflected ultrasonic signals comprises an ultrasonic Doppler blood flow detector whose piezoelectric transmitting and receiving elements are semicircular discs or rectangles positioned in the space provided between the light emitting diodes.

Preferably, the piezoelectric elements are attached to the optically and ultrasonically transparent window overlying the light emitting diodes.

Preferably, the apparatus further comprises a switching device to which the said means for applying light and ultrasound to the tissues being interrogated can be selected. When the optical system comprising the D-shaped array of light emitting diodes reveals an area of darkness on the superior aspect of the breast to the user caused by optical absorption in the tissues, the switch may be moved to permit ultrasonic interrogation of that region of the tissue giving rise to the absorption.

Preferably, the time varying Doppler frequency shift which is electronically produced is revealed to the user via an audio system such as internal or external loudspeaker or headphones.

Preferably, the combined optical ultrasound Doppler device includes an array of two or more piezoelectric elements for blood flow detection and analysis, operating at ultrasonic frequencies between 2 and 20 MHz.

Preferably, the intensity of the optical radiation is electronically incremented in steps which are indicated digitally or otherwise to the user so that serial examinations lead to images of tissues comparison of which permits the user to spot changes to the appearance of the illuminated tissue surface caused by underlying pathology at an early stage in the disease process.

In the invention, the light level is held at a low level until a pressure or capacitive sensor incorporated in the device touches the skin whereupon the light level increases to operate at the preset level thus protecting the eyes of the user and allowing them to maintain visual sensitivity.

The device may be protected from overheating by a heat sink situated behind the light emitting diodes and an embedded thermostatic element which at a selected temperature interrupts the current driving the diodes.

Preferably, the optical features are included but the ultrasonic features are disabled or omitted.

Preferably, the device has an output socket connectable to an external recording system able to present an ultrasonic Doppler frequency shift spectra.

Preferably, the apparatus is adapted to the examination of testicular tissues.

Preferably, contrast media are injected into the vascular system to increase the sensitivity of the Doppler ultrasound and optical interrogation of tissues.

Preferably, transparent gel or lubricant for enhancing optical penetration of tissues is applied between the window and the skin and through which light and ultrasound are transmitted.

Preferably, the optically transparent window has a light emitting face substantially in the shape of an angular segment of a circle.

Preferably, the light emitting face is substantially semi-circular, more preferably substantially D shaped.

Preferably, the light emitting face comprises a window which is transparent to radiation emitted from the light source.

The apparatus may further comprise a means for applying ultrasound to a neoplasm to be examined by the apparatus, a means for receiving reflected ultrasound from blood vessels in the tissues, and a means for generating an output to be analysed by a user which reproduces blood flow signals derived from the reflected ultrasound signals.

Preferably, the means for applying ultrasound and the means for receiving reflected ultrasound are positioned within the area of the light emitting face, more preferably within a centre of the light emitting face.

Preferably, the means for applying ultrasound and means for receiving reflected ultrasound comprises an ultrasonic Doppler blood flow detector having piezoelectric transmitting and receiving elements positioned in a space provided between the light emitting diodes.

Preferably, the piezoelectric elements are attached to the window overlying the light emitting diodes.

The apparatus may further comprise a switching device to which the said means for applying light and ultrasound to the tissues being interrogated can be selected.

The apparatus may further comprise an audio system by means of which a time varying Doppler frequency shift which is electronically produced is revealed to the user.

Preferably, the means for applying ultrasound and the means for receiving reflected ultrasound includes an array of two or more piezoelectric elements for blood flow detection and analysis, operating at ultrasonic frequencies between 2 and 20 MHz.

The apparatus may further comprise a controller for the light source whereby the intensity of the optical radiation is electronically incremented in steps which are indicated to the user.

The apparatus may further comprise a light level controller whereby the light level is held at a low level until the pressure or capacitive sensor incorporated in the device touches the skin whereupon the light level increases to operate at the preset level.

The apparatus may further comprise a heat sink situated behind the light emitting diodes and an embedded thermostatic element which at a selected temperature interrupts the current driving the diodes to protect the apparatus from overheating.

The apparatus may further comprise an output socket connectable to an external recording system able to present an ultrasonic Doppler frequency shift spectra.

The apparatus may further comprise a housing for the light source, the light emitting face having a linear edge adjacent to a linear side of the housing and a curved edge projecting away from the linear edge and located inwardly of a side of the housing, whereby the linear edge can be placed substantially against the skin of a user beneath the breast and substantially against the chest wall for illuminating the breast.

Preferably, the linear edge is about 5mm inwardly of the linear side of the housing.

Preferably, the housing has an integral handle portion remote from the light emitting face.

The present invention also relates to the use of the apparatus according to the present invention for the examination of breast or testicular tissues.

Preferably, contrast media are injected into the vascular system to increase the sensitivity of the Doppler ultrasound and optical interrogation of tissues.

Preferably, transparent gel or lubricant for enhancing optical penetration of tissues is applied between the window and the skin and through which light and ultrasound are transmitted.

Embodiments of the present invention will now be described by ay of example only with reference to the accompanying drawings, in which:-
Figure 1 shows an advanced cancer of the breast (arrowed) obtained by means of light transmitted through the tissues from a known light guide held against the inferior breast surface;
Figure 2 shows a first arrangement of 8 LEDs, not in accordance with the present invention, used to demonstrate the possibility of illuminating breast tissues from the inferior aspect with these multiple light sources;
Figure 3 shows an image of a breast obtained using apparatus shown in Figure showing serious backscatter of light below and around the periphery of the breast;

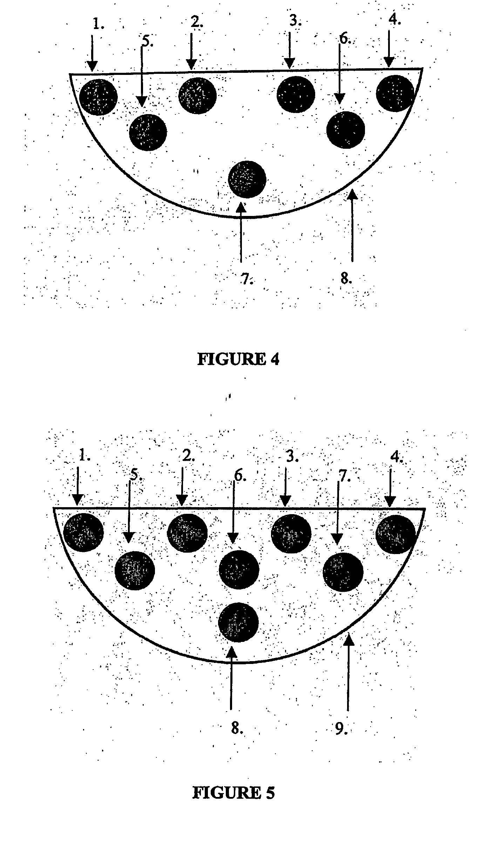

Figure 4 shows an array of light emitting diodes in D formation incorporated in an apparatus for examining a body of living tissues in accordance with a first embodiment of the present invention;
Figure 5 shows an array of light emitting diodes in D formation incorporated in an apparatus for examining a body of living tissues in accordance with a second embodiment of the present invention;

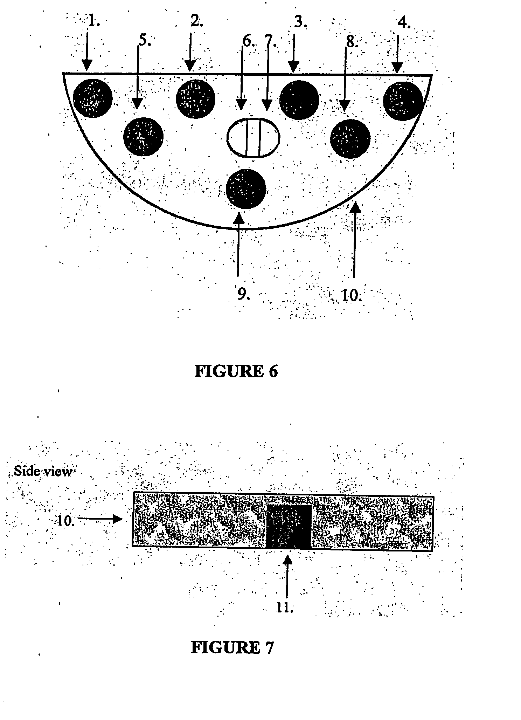

Figure 6 shows an array of light emitting diodes in D formation incorporated in an apparatus for examining a body of living tissues in accordance with a third embodiment of the present invention;
Figure 7 shows an optically transparent window which covers the LED array having a circular recess into which the piezoelectric transducers are fixed for incorporation into any of the embodiments of the present invention;

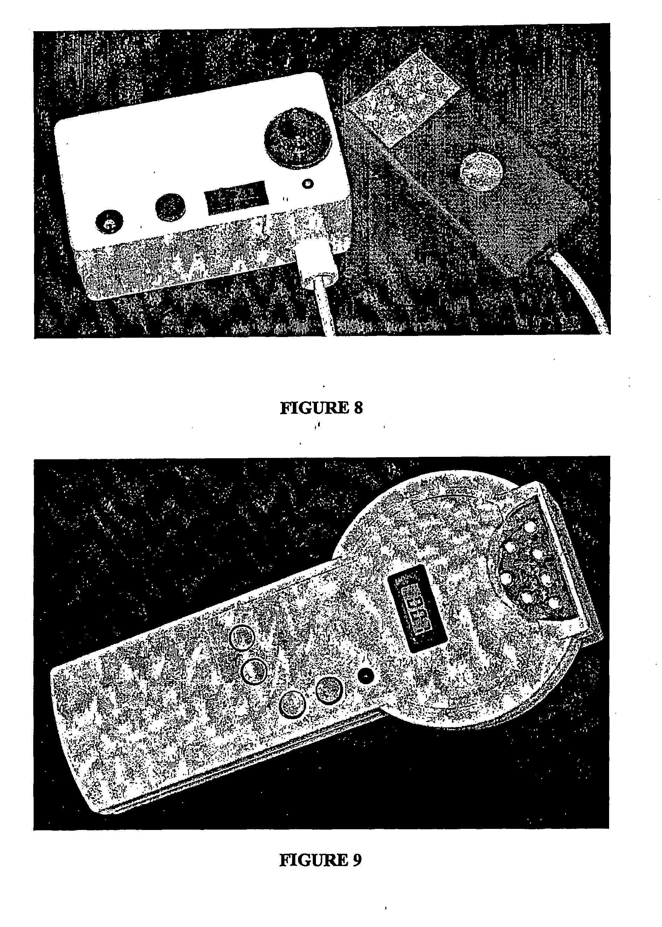

Figure 8 show an apparatus in accordance with a fourth embodiment of the present invention with a rectangular window including 7 LEDs in D shaped configuration;
Figure 9 shows a hand held breast checker in accordance with a fifth embodiment of the present invention with 7 LEDs in D-shaped array with D-shaped transparent window;

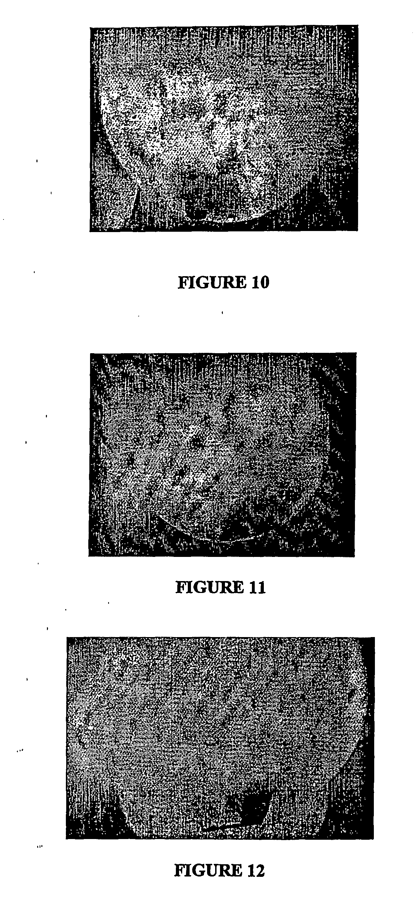

Figures 10 and 11 are images of right (R) and left (L) breasts obtained using the apparatus of Figure 8;

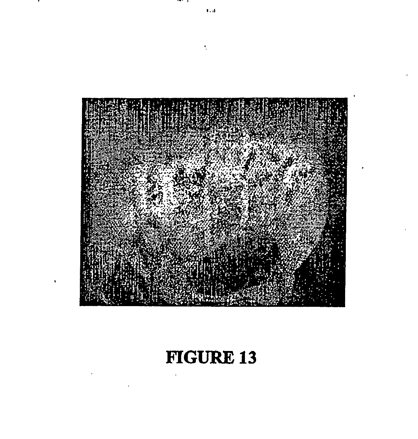

Figures 12 and 13 are images of right (R) and left (L) breasts obtained using the apparatus of Figure 9;

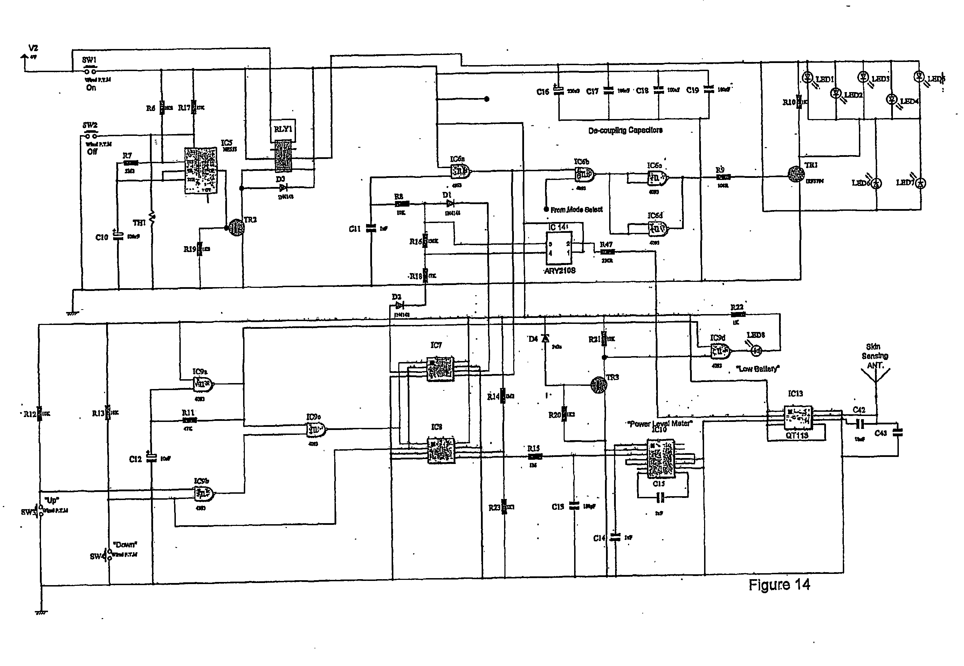

Figure 14 shows circuitry that controls the LEDs in the device shown in Figure 9;

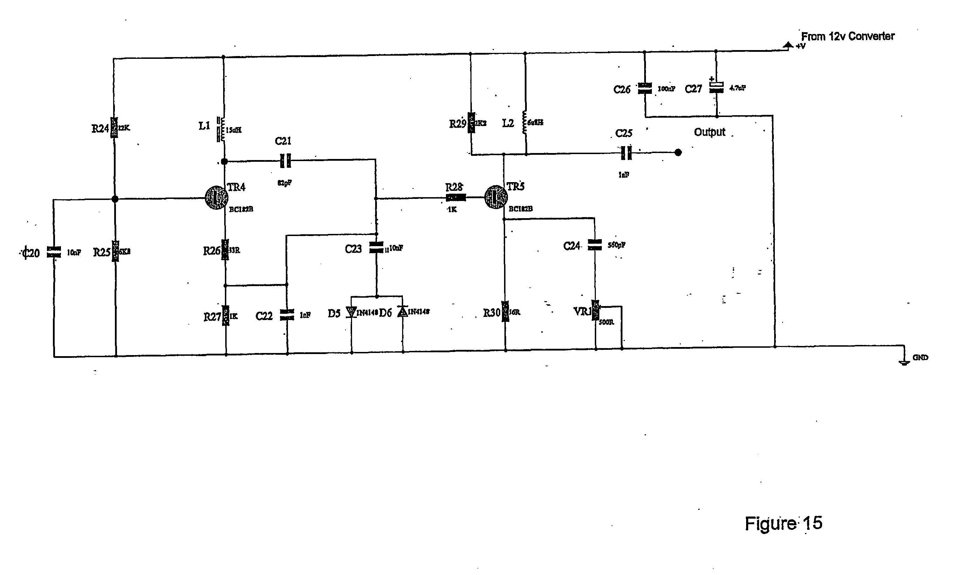

Figure 15 shows circuitry of the Doppler ultrasound transmitter in the device shown in Figure 9; and

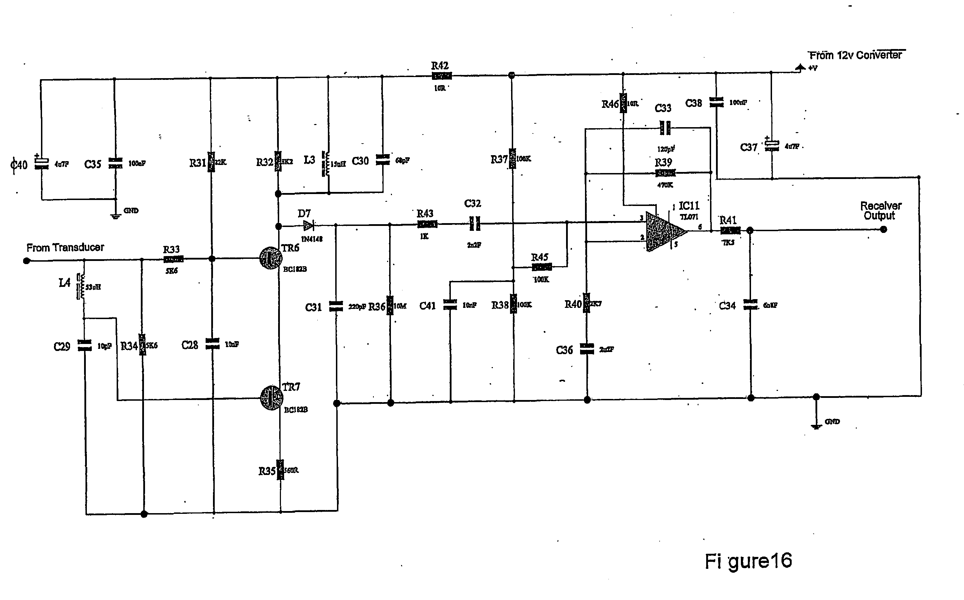

Figure 16 shows circuitry of the Doppler ultrasound receiver in the device shown in Figure 9.

Experiments demonstrated that a D-shaped array of LEDs, as shown in the apparatus of Figure 4, dramatically reduced this problem. To increase the intensity of light transmitted through the breast tissues 8 LEDs can be used as shown in Figure 5. and still retain the D configuration.

Figure 4 shows 7 light emitting diodes in D formation. The preferred dimensions are 45 mm chord length and 20 mm in width. LEDs arrowed 1-7. 8 transparent window.

Figure 5 shows 8 light emitting diodes in a D formation. The preferred dimensions are 45 mm along the chord and 20 mm in width. LEDs arrowed 1-8. Transparent window 9.

Figure 6 shows a similar arrangement of LEDs to that of Figure 4 but 2 D shaped piezoelectric elements are diameter around 8 mm labelled 6 and 7, inserted in the window above the LED array. Connecting wires to these transducers are now shown. LEDs arrowed 1,2,3,4,5,8 and 9. Transparent window 10.

Figure 7 shows an optically transparent window which covers the LED array having a circular recess into which the piezoelectric transducers are fixed. The layer of material above the transducers to which these elements are attached is minimised to around typically 0.2 mm. Window is labelled 10 and the well for housing the piezoelectric transducer elementsis labelled 11.

In course of our experiments we also demonstrated the need for a row of LEDs to be placed close to the chest wall to ensure useful examination of the entire breast volume. The separation of 10 mm shown in Figure 2 is too great and was reduced to about 5 mm in the apparatus shown in Figure 6.

Another advantage of the D-shaped array of LEDs is the ability to include piezoelectric transducers as shown in Figure 6 without reducing the light transmitted into tissues. One piezoelectric element transmits ultrasound [pulsed or continuous wave] into breast tissues. Where there is a cancer and associated angiogenesis the sound is scattered by red blood cells and this scattered sound is received by the second piezoelectric element The frequency shift df of the ultrasonic wave contains information about the blood flow velocities in the angiogenesi vessels and associated arterio venous shunts. Note that df = 2v f cos q/c where v is the flow velocity, f is the transmitted frequency, c is the speed of sound and q is the angle between flow direction and that of the interrogating wave. Our preferred method of including the transducers is to remove a portion of the transparent window (10) which encloses the LEDs. This cavity is numbered 11 in Figure 7. The preferred thickness of the residual window is 0.2 mm to minimise ultrasonic absorption in the material comprising the window. The transducers may be glued onto the upper face of the cavity which is typically 8 mm in diameter. The rear face of the transducers is preferably air backed for maximum sensitivity though a tungsten loaded epoxy based material may be used as backing material. The preferred frequency of the ultrasound is between 2 and 15 MHz. Lower frequencies ensure sufficient penetration of tissues to reach typical tumours.

A further advantage of the invention described here is that the transducers and associated electronics can be operated while light is passing through tissues. This means that the ultrasonic waves can be directed at the tumour, guided by its optical image, to determine whether or not a tumour is vascularised. The mains operated versions prove more difficult in this respect because electrical noise generated by the fan [referred to above] 'over power' the Doppler ultrasound signals. The Doppler signals in the audio frequency range may be extracted electronically, amplified and presented via a mini-loudspeaker in the unit. Characteristic pulsatile sounds from a tumour can be heard by the user when present. Such signals are another indicator of the need to seek medical advice. Note that coupling gel such as KY gel placed on the window does not inhibit imaging but enhances the ultrasonic aspect of the examination.

Figure 8 show the apparatus with a rectangular window including 7 LEDs in D shaped configuration. The (white) unit connected to the array contains pulse width modulation electronic circuitry and a calibrated control [black knob with scale] to allow repeatability of the light intensity used in one examination to be reproduced in the next. The system is operated with rechargeable batteries.

Figure 9 shows a hand held breast checker with 7 LEDs in D-shaped array with D-shaped transparent window. There are facilities to increment the light intensity in typically 20 steps up and 20 steps down from the 50% starting level. There is a digital display to show the light level. A memory chip can reset the unit to give the same intensity at next use. A low battery warning light flashes at a predetermined voltage, typically after 30 minutes use. Undue internal heating from prolonged use of the device is obviated by a thermal cut out set at typically 60 degrees Centigrade. Note the need for a very narrow separation between the position of the 4 LEDs [in a line] and the terminal end of the instrument which is normally placed next to the chest wall when in use.

In Figure 9 a light only unit is shown with 7 LEDs configured in a D-shape. There are on and off buttons plus buttons to increase and decrease the light intensity for optimum light intensity which is not normally maximum intensity. Failure to appreciate this latter point can cause scattering around a tumour to a degree which suppresses the tumour generated optical absorption thus foiling the objective of using the device. The D shaped window and LEDs in Figure 7 is arranged to be as close as possible to the chest wall of a user. In the preferred device, not shown, the window is curved to fit the curvature of a typical chest wall and for use by doctors or other clinical personnel the Doppler system is included within the hand held device. The unit shown in Figure 9 contains 3 AA batteries, runs for over 30 minutes and is supplied with a separate charger unit.

In the preferred unit the red LEDs operate at about 630 nanometres. Red/orange and Amber LEDs [617 and 590 nm respectively] may be used but the tissues are less transparent at those wavelengths and higher light intensities maybe required to produce useful breast images.

There is a low battery warning light indicating the need for connection to recharging circuitry. An LCD display or stack of low power LEDs shows the light intensity on a scale of 0-100%. A facility to disable the breast checker while being recharged from the mains is desirable and is included in the preferred embodiment.

Figures 10 and 11 are images of R and L breasts obtained using the apparatus of Figure 8. Superficial blood vessels, areola and nipples are demonstrated and only a modest light component is scattered laterally and there is virtually non in the forward direction.

Figure 12 shows how increasing the light transmission through tissues beyond optimum decreases contrast so that the blood vessels become barely visible. Figure 13 shows an image produced by the apparatus described in Figure 9 and demonstrates excellent contrast between blood vessels and tissue, however there is forward light scatter because of the depression in the casing next to the D-shaped window. This depression is omitted in the preferred instrument outlined in this patent application.

The clarity of superficial vessels is a marker for obtaining good images of angiogenesis at a depth in tissue.

Figure 10 shows an image of R breast obtained with apparatus of Figure 8.

Figure 11 shows an image of L breast obtained with apparatus of Figure 8.

Figure 12. Image to show over exposed image ofR breast with light of intensity set at a level which is too high. Neoplastic tumours will not show up in these circumstances. The apparatus shown in Figure 9 was used.

Figure 13. Image of L breast obtained with apparatus of Figure 9 set at the correct intensity level.

The sensitivity of more primitive earlier versions of the apparatus described here demonstrated sensitivity for detecting cancer of 0.94 and 0.73 Bundred et al (1986) and Brittenden et al (1995). One of the present inventors first showed that light passing through normal and pathological breast tissue samples demonstrated cancer because of associated angiogenesis, Watmough (1982). Thus when a cancer is demonstrated by the breast checker, there is angiogenesis. It follows that the cancer is in its exponentially growing phase and is therefore life threatening. Some microscopic cancers without angiogenesis may be dormant and never surface clinically in the patient's life time. This fact emphasizes the value of the breast checker since microscopic cancers without angiogenesis will fail to be revealed. This is a strength rather than a weakness.

The preferred LEDs used to obtain the images presented here operate at 627 nanometres but similar devices which operate at 617 and 590 nanometres can be employed and even combinations within one optical or optical/Doppler probe. Typical electrical power rating is 1 Watt per LED but employment of higher power devices are envisaged. Any LED with wavelength between about 550 nm and about 700 nm can in principle be employed.

The components and electronic circuitry included in the units described here are shown by way of example only in Figures 14-16. The circuitry shown in Figure 14 controls the LEDs in the device shown in Figure 9. The circuitry seen in Figure 15 describes the Doppler ultrasound transmitter and Figure 16 that of the receiver. The power amplifier and loudspeaker are not shown here. These circuits and the particular dimensions described in this document are to be treated as examples useful for implementing the invention. The transducer elements may be semi circular as described here or can be two rectangular slabs of suitable dimensions. The two piezoelectric elements maybe subdivided into, for example, 4 segments, opposite quadrants being connected together. This latter can reduce the effect of vessel orientation with respect to the breast checker geometry on Doppler signal detection.

The thickness of the piezoelectric elements determines their operating frequency, normally being half wavelength.

According to the invention described there is a microprocessor which allows reset of the light levels to the previous value to facilitate serial breast examinations, controls current through the LEDs, warns of low battery level and in the case of the clinical version of the breast checker interprets the Doppler signals in terms of advice to go for medical assessment. This can be a flashing light or a message on an LCD display. The microprocessor in the breast checker allows the light intensity from the probe to be stepped up automatically to the reset level in order to realise optimum contrast for each user. The reset level refers to intensity employed at last use the device. The device shuts down after a predetermined time.

According to the invention a capacitive [or mechanical] microswitch maintains low light level until the breast checker is in contact with the skin. This feature protects the eyes of users which might otherwise develop temporarily reduced visual sensitivity after viewing bright LEDs.

In the preferred embodiment a sensor to detect ambient light level and adjust LED brightness accordingly can be included.

It is likely that patients with locally advanced breast cancer might be monitored to demonstrate the efficacy of chemotherapy or angiogenesis inhibiting drugs on their tumour using the breast checker described in this application.

### References.

Bundred N., Levack P., Watmough D.J., and Watmough J.A. [1986]. Preliminary results using computerised tele-Diaphanography for the investigation of breast disease. British J. Hospital Med. 37, 70-71.
Brittenden J, Watmough D J, Heys S D and Eremin O. 1995 Preliminary clinical evaluation of a combined optical Doppler ultrasound instrument for the detection of breast cancer. Brit. J Radiology 68, 1344 - 1348.
Watmough D.J. [1982]. Diaphanography; Mechanism responsible for the images. Acta. Radiologica Oncol. 21, 11-15.

## Claims

1. A handheld battery powered apparatus for examining a body of living tissues, the apparatus comprising:
a light source for illuminating the tissues;
the light source comprising a substantially D shaped array of high intensity light emitting diodes (1-7;1-8;1-5,8,9) overlaid by an optically transparent window (8,9;10) wherein said light emitting diodes (1-7;1-8;1-5,8,9) operate at a wavelength lying between 550 and 700 nm;
a control means for reproducing the intensity of the light from the light source operarable to allow repeatability of the light intensity used in one examination to be reproduced in the next;
**characterised in that**
the apparatus comprises a pressure or capacitive sensor whereby the light level is held at a low level until the pressure or capacitive sensor touches the skin of the body of tissues whereupon the light level increases to operate at a preset level of light intensity.

2. An apparatus according to any preceding claim in which the control means is adapted whereby the intensity of the light is electronically incremented in steps which are indicated digitally or otherwise to the user so that serial examinations lead to images of tissues, comparison of which permits the user to spot changes to the appearance of the illuminated tissue surface caused by underlying pathology at an early stage in the disease process.

3. An apparatus according to any preceding claim in which the optically transparent window has a light emitting face (8;9;10) substantially in the shape of an angular segment of a circle.

4. An apparatus according to claim 3, wherein the light emitting face is substantially semi-circular.

5. An apparatus according to claim 3 or 4, in which the light emitting face is substantially D shaped.

6. An apparatus according to any preceding claim further comprising a housing for the light source, the light emitting face having a linear edge adjacent to a linear side of the housing and a curved edge projecting away from the linear edge and located inwardly of a side of the housing, whereby, in use, the linear edge can be placed substantially against the skin of a user beneath a breast and substantially against a user's chest wall for illuminating the breast.

7. An apparatus according to claim 6 wherein the linear edge is about 5mm inwardly of the linear side of the housing.

8. An apparatus according to preceding claims 6 or 7 wherein the apparatus comprises a housing and the housing has an integral handle portion remote from the optically transparent window having the light emitting face.

9. An apparatus according to any preceding claim comprising means (6) for applying ultrasound to a neoplasm to be examined, means (7) for receiving reflected ultrasound from blood vessels in the tissues, and a loudspeaker or other means for generating an output to be analysed by a user which reproduces blood flow signals derived from the reflected ultrasound signals.

10. An apparatus according to any preceding claim wherein the means for applying ultrasound and means for receiving reflected ultrasonic signals comprises an ultrasonic Doppler blood flow detector having piezoelectric transmitting and receiving elements (6,7) positioned in a space provided between the light emitting diodes (1-7;1-8;1-5,8,9).

11. An apparatus according to claim 10 wherein the piezoelectric transmitting and receiving elements (6,7) are semicircular discs or rectangles.

12. An apparatus according to claim 10 or 11 wherein the piezoelectric elements (6, 7) are attached to the optically and ultrasonically transparent window (8,9;10) overlying the light emitting diodes (1-7;1-8;1-5,8,9).

13. An apparatus according to any of claims 9 to 12 wherein the means (6) for applying ultrasound and the means (7) for receiving reflected ultrasound are positioned within the area of the light emitting face (8;9;10).

14. An apparatus according to claim 13, wherein the means for applying ultrasound and the means for receiving reflected ultrasound are positioned within a centre of the light emitting face.

15. An apparatus according to any one of claims 9 to 14 wherein the means (6) for applying ultrasound and means (7) for receiving reflected ultrasound include an array of two or more piezoelectric elements (6,7) for blood flow detection and analysis, which operate at ultrasonic frequencies between 2 and 20 MHz.

16. An apparatus according to any of claims 9 to 15 comprising a switching device for switching between the means for applying light and ultrasound to the tissues.

17. An apparatus according to any preceding claim further comprising a heat sink situated behind the light emitting diodes (1-7;1-8;1-5,8,9) and an embedded thermostatic element which at a selected temperature interrupts the current driving the diodes to protect the apparatus from overheating.

18. An apparatus according to any of claims 9 to 17 in which the ultrasonic features are disabled.

19. An apparatus according to any preceding claim in which 7 or 8 LEDs are provided.

## Patentansprüche

1. Tragbare Vorrichtung mit Batterieversorgung zum Untersuchen eines Körpers aus lebenden Geweben, wobei die Vorrichtung Folgendes umfasst:
eine Lichtquelle zum Beleuchten der Gewebe;
wobei die Lichtquelle eine im Wesentlichen D-förmige Anordnung von Leuchtdioden (1-7; 1-8; 1-5, 8, 9) mit hoher Intensität enthält, denen ein optisch durchlässiges Fenster (8, 9; 10) überlagert ist, wobei die Leuchtdioden (1-7; 1-8; 1-5, 8, 9) bei einer Wellenlänge im Bereich von 550 bis 700 nm arbeiten;
ein Steuermittel zum Reproduzieren der Stärke des Lichts von der Lichtquelle, das betreibbar ist, um eine Wiederholbarkeit der bei einer verwendeten Lichtstärke zu ermöglichen, um sie in der nächsten Untersuchung zu reproduzieren;
**dadurch gekennzeichnet, dass**
die Vorrichtung einen Druck- oder Kapazitätssensor umfasst, wobei der Lichtpegel auf einem niedrigen Pegel gehalten wird, bis der Druck- oder Kapazitätssensor die Haut des Körpers aus Geweben berührt, woraufhin der Lichtpegel zunimmt, um mit einem im Voraus festgelegten Lichtstärkepegel zu arbeiten.

2. Vorrichtung nach einem vorhergehenden Anspruch, wobei das Steuermittel dafür ausgelegt ist, die Stärke des Lichts elektronisch in Schritten zu inkrementieren, die dem Anwender digital oder auf andere Weise angezeigt werden, so dass Reihenuntersuchungen zu Bildern von Geweben führen, deren Vergleich dem Anwender ermöglicht, Änderungen des Erscheinungsbildes der beleuchteten Gewebeoberfläche, die durch eine darunterliegende Pathologie verursacht werden, auf einer frühen Stufe des Erkrankungsprozesses zu erkennen.

3. Vorrichtung nach einem vorhergehenden Anspruch, wobei das optisch durchlässige Fenster eine Lichtemissionsfläche (8; 9; 10) besitzt, die im Wesentlichen die Form eines Kreiswinkelsegments hat.

4. Vorrichtung nach Anspruch 3, wobei die Lichtemissionsfläche im Wesentlichen halbkreisförmig ist.

5. Vorrichtung nach Anspruch 3 oder 4, wobei die Lichtemissionsfläche im Wesentlichen D-förmig ist.

6. Vorrichtung nach einem vorhergehenden Anspruch, die ferner ein Gehäuse für die Lichtquelle umfasst, wobei die Lichtemissionsfläche eine geradlinige Kante in der Nähe einer geradlinigen Seite des Gehäuses und eine gekrümmte Kante, die von der geradlinigen Kante vorsteht und sich innerhalb einer Seite des Gehäuses befindet, besitzt, wobei im Gebrauch die geradlinige Kante im Wesentlichen an die Haut eines Anwenders unterhalb einer Brust und im Wesentlichen an eine Brustkorbwand des Anwenders angelegt werden kann, um die Brust zu beleuchten.

7. Vorrichtung nach Anspruch 6, wobei die geradlinige Kante sich ungefähr 5 mm innerhalb der geradlinigen Seite des Gehäuses befindet.

8. Vorrichtung nach den vorhergehenden Ansprüchen 6 oder 7, wobei die Vorrichtung ein Gehäuse umfasst und das Gehäuse einen einteiligen Griffabschnitt in einem Abstand von dem optisch durchlässigen Fenster, das die Lichtemissionsfläche besitzt, aufweist.

9. Vorrichtung nach einem vorhergehenden Anspruch, die Mittel (6), um auf ein zu untersuchendes Neoplasma Ultraschall anzuwenden, Mittel (7), um reflektierten Ultraschall von Blutgefäßen in den Geweben zu empfangen, und einen Lautsprecher oder andere Mittel, um einen von einem Anwender zu analysierenden Ausgang zu erzeugen, der Blutstromsignale, die aus den reflektierten Ultraschallsignalen abgeleitet werden, reproduziert, umfasst.

10. Vorrichtung nach einem vorhergehenden Anspruch, wobei die Mittel zum Anwenden von Ultraschall und die Mittel zum Empfangen reflektierter Ultraschallsignale einen Ultraschall-Doppler-Blutstromdetektor umfassen, der piezoelektrische Sende- und Empfangsmittel (6, 7) besitzt, die in einem Raum positioniert sind, der zwischen den Leuchtdioden (1-7; 1-8; 1-5, 8, 9) vorgesehen ist.

11. Vorrichtung nach Anspruch 10, wobei die piezoelektrischen Sende- und Empfangsmittel (6, 7) halbkreisförmige Scheiben oder Rechtecke sind.

12. Vorrichtung nach Anspruch 10 oder 11, wobei die piezoelektrischen Elemente (6, 7) an dem für Licht und Ultraschall durchlässigen Fenster (8, 9; 10), das über den Leuchtdioden (1-7; 1-8; 1-5, 8, 9) liegt, befestigt sind.

13. Vorrichtung nach einem der Ansprüche 9 bis 12, wobei die Mittel (6) zum Anwenden von Ultraschall und die Mittel (7) zum Empfangen von reflektiertem Ultraschall in dem Bereich der Lichtemissionsfläche (8; 9; 10) positioniert sind.

14. Vorrichtung nach Anspruch 13, wobei die Mittel zum Anwenden von Ultraschall und die Mittel zum Empfangen von reflektiertem Ultraschall in einem Zentrum der Lichtemissionsfläche positioniert sind.

15. Vorrichtung nach einem der Ansprüche 9 bis 14, wobei die Mittel (6) zum Anwenden von Ultraschall und die Mittel (7) zum Empfangen von reflektiertem Ultraschall eine Anordnung aus zwei oder mehr piezoelektrischen Elementen (6, 7) für die Blutstromdetektion und -analyse, die mit Ultraschallfrequenzen im Bereich von 2 bis 20 MHz arbeiten, enthalten.

16. Vorrichtung nach einem der Ansprüche 9 bis 15, die eine Schaltvorrichtung umfasst, um zwischen den Mitteln zum Anwenden von Licht und von Ultraschall auf die Gewebe umzuschalten.

17. Vorrichtung nach einem vorhergehenden Anspruch, die ferner einen Kühlkörper, der sich hinter den Leuchtdioden (1-7; 1-8; 1-5, 8, 9) befindet, und ein eingebettetes thermostatisches Element, das bei einer ausgewählten Temperatur den die Dioden ansteuernden Strom unterbricht, umfasst, um die Vorrichtung vor einer Überhitzung zu schützen.

18. Vorrichtung nach einem der Ansprüche 9 bis 17, wobei die Ultraschallmerkmale deaktiviert sind.

19. Vorrichtung nach einem vorhergehenden Anspruch, wobei 7 oder 8 LEDs vorgesehen sind.

## Revendications

1. Appareil à main alimenté par une batterie pour examiner une masse de tissus vivants, l'appareil comprenant :
une source de lumière pour éclairer les tissus ;
la source de lumière comprenant un réseau sensiblement en forme de D de diodes électroluminescentes de forte intensité (1-7 ; 1-8 ; 1-5, 8, 9) recouvertes par une fenêtre optiquement transparente (8, 9 ; 10), lesdites diodes électroluminescentes (1-7 ; 1-8 ; 1-5, 8, 9) fonctionnant à une longueur d'onde se situant entre 550 et 700 nm ;
un moyen de commande pour reproduire l'intensité de la lumière provenant de la source de lumière utilisable pour permettre à la répétabilité de l'intensité lumineuse utilisée dans un examen d'être reproduite dans le suivant ;
**caractérisé en ce que**
l'appareil comprend un capteur de pression ou capacitif, le niveau de lumière étant maintenu à un niveau bas jusqu'à ce que le capteur de pression ou capacitif touche la peau de la masse de tissus, moment auquel le niveau de lumière augmente pour fonctionner à un niveau préréglé d'intensité lumineuse.

2. Appareil selon la revendication précédente dans lequel le moyen de commande est adapté pour que l'intensité de la lumière soit incrémentée électroniquement par paliers qui sont indiqués numériquement ou d'une autre manière à l'utilisateur de telle sorte que des examens en série aboutissent à des images de tissus dont une comparaison permet à l'utilisateur de repérer des changements d'apparence de la surface tissulaire éclairée causés par une pathologie sous-jacente à un stade précoce dans le processus pathologique.

3. Appareil selon l'une quelconque des revendications précédentes dans lequel la fenêtre optiquement transparente comporte une face émettant de la lumière (8 ; 9 ; 10) sensiblement sous la forme d'un segment angulaire d'un cercle.

4. Appareil selon la revendication 3 dans lequel la face émettant de la lumière est sensiblement semi-circulaire.

5. Appareil selon la revendication 3 ou 4 dans lequel la face émettant de la lumière est sensiblement en forme de D.

6. Appareil selon l'une quelconque des revendications précédentes comprenant en outre un logement pour la source de lumière, la face émettant de la lumière ayant un bord linéaire adjacent à un côté linéaire du logement et un bord incurvé s'éloignant du bord linéaire et situé à l'intérieur d'un côté du logement, d'où il résulte que, à l'usage, le bord linéaire peut être placé sensiblement contre la peau d'une utilisatrice en dessous d'un sein et sensiblement contre la paroi thoracique de l'utilisatrice pour éclairer le sein.

7. Appareil selon la revendication 6 dans lequel le bord linéaire se situe environ 5 mm à l'intérieur du côté linéaire du logement.

8. Appareil selon la revendication 6 ou 7 précédente, l'appareil comprenant un logement, et le logement comportant une partie de poignée intégrale distante de la fenêtre optiquement transparente comportant la face émettant de la lumière.

9. Appareil selon l'une quelconque des revendications précédentes comprenant un moyen (6) pour appliquer des ultrasons à un néoplasme à examiner, un moyen (7) pour recevoir les ultrasons réfléchis par les vaisseaux sanguins dans les tissus, et un haut-parleur ou un autre moyen pour générer une sortie à analyser par un utilisateur qui reproduit les signaux du débit sanguin dérivés des signaux ultrasonores réfléchis.

10. Appareil selon l'une quelconque des revendications précédentes dans lequel le moyen pour appliquer des ultrasons et le moyen pour recevoir les signaux ultrasonores réfléchis comprennent un détecteur de débit sanguin à effet Doppler ultrasonore comportant des éléments piézoélectriques d'émission et de réception (6, 7) positionnés dans un espace situé entre les diodes électroluminescentes (1-7 ; 1-8 ; 1-5, 8, 9).

11. Appareil selon la revendication 10 dans lequel les éléments piézoélectriques d'émission et de réception (6, 7) sont des disques semi-circulaires ou des rectangles.

12. Appareil selon la revendication 10 ou 11 dans lequel les éléments piézoélectriques (6, 7) sont fixés à la fenêtre transparente à la lumière et aux ultrasons (8, 9 ; 10) recouvrant les diodes électroluminescentes (1-7 ; 1-8 ; 1-5, 8, 9).

13. Appareil selon l'une quelconque des revendications 9 à 12 dans lequel le moyen (6) pour appliquer des ultrasons et le moyen (7) pour recevoir les ultrasons réfléchis sont positionnés à l'intérieur de la zone de la face émettant de la lumière (8, 9 ; 10).

14. Appareil selon la revendication 13 dans lequel le moyen pour appliquer des ultrasons et le moyen pour recevoir les ultrasons réfléchis sont positionnés au centre de la face émettant de la lumière.

15. Appareil selon l'une quelconque des revendications 9 à 14 dans lequel le moyen (6) pour appliquer des ultrasons et le moyen (7) pour recevoir les ultrasons réfléchis comprennent un réseau d'au moins deux éléments piézoélectriques (6, 7) pour détection et analyse du débit sanguin, qui fonctionnent à des fréquences ultrasonores comprises entre 2 et 20 MHz.

16. Appareil selon l'une quelconque des revendications 9 à 15 comprenant un dispositif de commutation pour basculer entre les moyens pour appliquer de la lumière et des ultrasons aux tissus.

17. Appareil selon l'une quelconque des revendications précédentes comprenant en outre un dissipateur thermique situé derrière les diodes électroluminescentes (1-7 ; 1-8 ; 1-5, 8, 9) et un élément thermostatique intégré qui, à une température sélectionnée, coupe le courant alimentant les diodes pour protéger l'appareil d'une surchauffe.

18. Appareil selon l'une quelconque des revendications 9 à 17 dans lequel les fonctions ultrasonores sont désactivées.

19. Appareil selon l'une quelconque des revendications précédentes dans lequel 7 ou 8 DEL sont présentes.
